# EUROPEAN PATENT SPECIFICATION

(11) **EP 2 526 913 B1**
(45) Date of publication and mention of the grant of the patent: **12.10.2016**
(21) Application number: 11734607.2
(22) Date of filing: 17.01.2011
(51) Int. Cl.: A61F 13/496

(54) **PULL-UP DISPOSABLE WEARING ARTICLE**
EINWEGBEKLEIDUNGSARTIKEL ZUM HOCHZIEHEN
ARTICLE CULOTTE PORTABLE JETABLE

(30) Priority: 21.01.2010 JP 2010011309
(43) Date of publication of application: 28.11.2012
(73) Proprietor: Unicharm Corporation, Ehime 799-0111 (JP)
(72) Inventor: YAMASHITA, Mariko, Kanonji-shi Kagawa 769-1602 (JP); OTSUBO, Toshifumi, Kanonji-shi Kagawa 769-1602 (JP); HASHIMOTO, Tatsuya, Kanonji-shi Kagawa 769-1602 (JP); KAMIYAMA, Ryuichi, Kanonji-shi Kagawa 769-1602 (JP)
(74) Representative: Dolleymores
(86) International application number: PCT/JP2011/050665
(87) International publication number: WO 2011/090001

(56) References cited:
- JP-A- 2002 272 785
- JP-A- 2003 038 572
- JP-A- 2003 339 772
- JP-A- 2003 339 772
- JP-A- 2005 287 930
- JP-A- 2006 006 780
- JP-A- 2009 131 539
- JP-U- H0 631 721
- US-A1- 2007 073 261

## Description

### {Technical Field}

The present invention relates to pants-type disposable wearing articles and, more particularly, to pants-type disposable wearing articles such as disposable diapers, disposable toilet-training pants, disposable incontinent pants, disposable sanitary pants and the like, respectively of pants-type, wherein front and rear waist regions are joined together along respective side edges thereof by side seams each including a plurality of sealing spots.

### {Background}

Conventionally, pants-type disposable wearing articles provided with the side seams adapted to join the front and rear waist regions together along side edges of the respective waist regions are known. For example, JP 2008-136651 A (PTL 1) discloses a pants-type disposable wearing article having a front waist region, a rear waist region, a crotch region extending between the front and rear waist regions and side seams provided along the side edges of the front and rear waist regions, each of the side seams including a plurality of sealing spots arranged intermittently in a longitudinal direction of the diaper.

Japanese patent application nos. JP 2009-131539 (PTL2) and JP 2003-339772 (PTL3) disclose a pants type disposable diaper, and US patent application no. US 2007/073261 is directed towards a side seam for a disposable garment.

### {Citation List}

### {Patent Literature}

{PTL 1} JP 2008-136651 A
[PTL 2] JP 2009-131539 A
[PTL 3] JP 2003-339772 A
{PTL 4] US 2007/073261 A1

### {Summary of Invention}

### {Technical Problem}

In the pants-type wearing article disclosed in PTL 1, the sealing spot having a smallest width dimension (i.e., a narrowest sealing spot) is positioned at the midpoint of the side seam between the top and bottom segments thereof so that a seam width of the sealing spot may be gradually reduced from the top and bottom segments toward the narrowest sealing spot. With such an arrangement, after the diaper has been used, the front and rear waist regions may be smoothly torn off each other merely by tearing one of the top and bottom segments since the top and bottom segments have a given joint strength but the joint strength of the intermediate segment is relatively low.

An object of the present invention is to provide a pants-type disposable wearing article wherein each of the side seams has a desired joint strength and the joint should not unintentionally be released particularly in the bottom segment during use of the wearing article.

### {Solution to Problem}

The present invention therefore provide the pants-type disposable wearing article of independent Claim 1. The dependent claims specify preferred but optional features. According to the present invention, there is provided a pants-type disposable wearing article having a longitudinal axis and a transverse axis being orthogonal thereto, and including a front waist region, a rear waist region, a crotch region extending between the front and rear waist regions, a waist-opening and side seams by which the front and rear waist regions are joined together along side edges thereof.

According to the present invention, the side seams are respectively formed of a plurality of sealing spots arranged intermittently in a direction along the longitudinal axis and include a top segment located adjacent to the waist-opening and a bottom segment located adjacent to the crotch region, and each of the sealing spots has a substantially uniform area and a dimension of the bottom segment in a direction along the transverse axis is larger than a dimension of the top segment in the direction along the transverse axis.

The present invention includes: (1) The side seams include an intermediate segment extending between the top segment and the bottom segment; the opposite side edges of the front and rear waist regions respectively include non-elasticized regions formed of a non-elasticized outer sheet, first elasticized regions lying below the non-elasticized regions and formed of the outer sheet and an elasticized inner sheet and second elasticized regions lying below the first elasticized regions and formed of the inner and outer sheets and stretchable leg elastic elements bonded to the inner and outer sheets; and the top segments are formed in the non-elasticized regions, the intermediate segments are formed in the first elasticized regions and the bottom segments are formed in the second elasticized regions.

The present invention further includes one or more of the following embodiments.
(2) A dimension of a side seam array in the direction along the longitudinal axis is gradually reduced in the bottom segment of the side seams from the outside toward the inside as viewed in the direction along the transverse axis.
(3) An area ratio of the sealing spots in the top segment formed in the non-elasticized region is lower than both an area ratio of the sealing spots in the intermediate segment formed in the first elasticized region and an area ratio of the sealing spots in the bottom segment formed in the second elasticized region.
(4) The sealing spots are arranged obliquely to the longitudinal axis and shaped so that a width dimension of the sealing spots is gradually enlarged in a direction of top-to-bottom.

### {Advantageous Effects of Invention}

In the wearing article according to the present invention, each of the sealing spots forming the respective side seams has the substantially uniform area and the dimension of the bottom segment in the direction along the transverse axis is larger than the dimension of the top segment in the direction along the transverse axis. With such an arrangement, the waist-opening has a size sufficient to facilitate the article to be handled and, at the same time, the peripheral edges of the leg-openings are sure to come in close contact with the wearer's skin at a desired fit. In addition, such a unique arrangement reliably eliminates the possibility that the sealing spots in the bottom segment might be unintentionally broken during use of the wearing article.

### {Brief Description of Drawings}

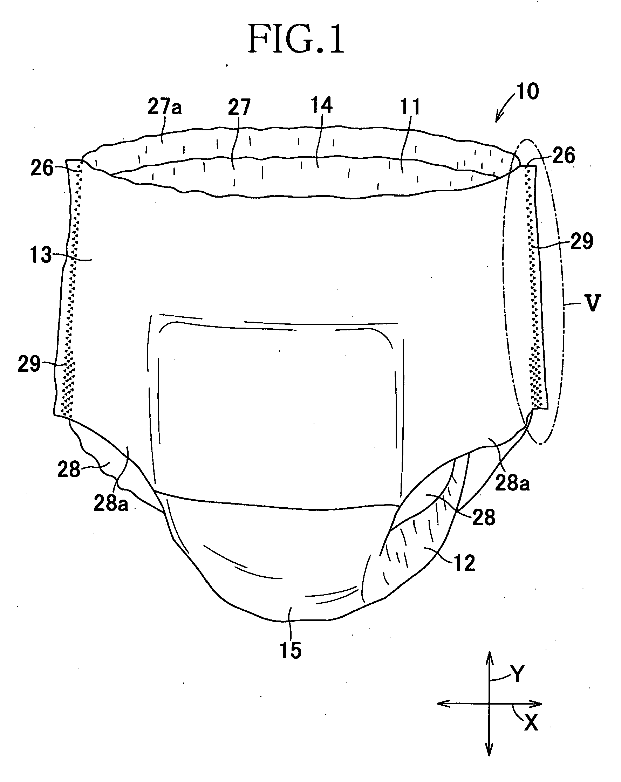

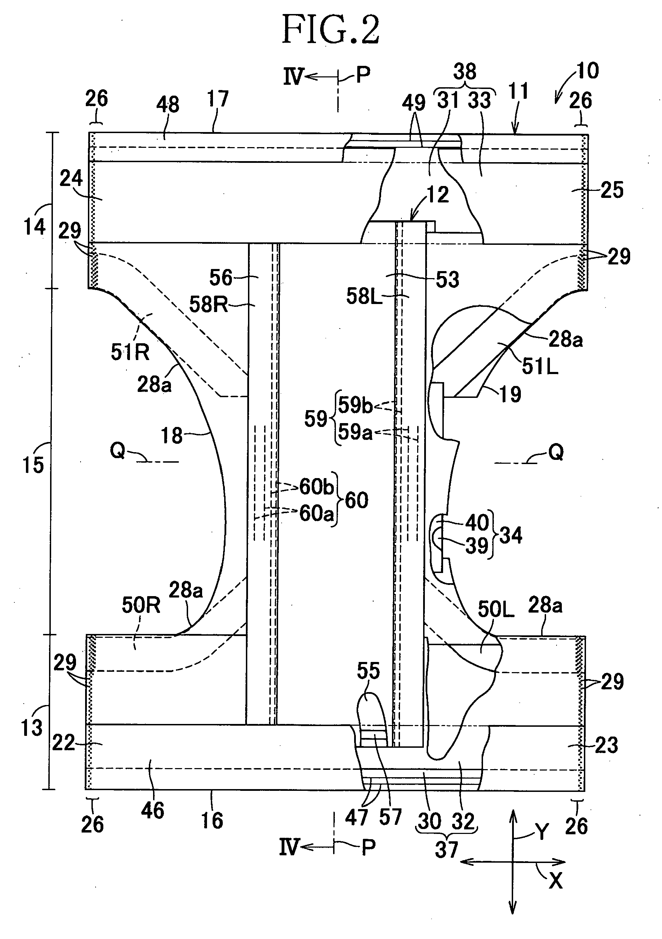

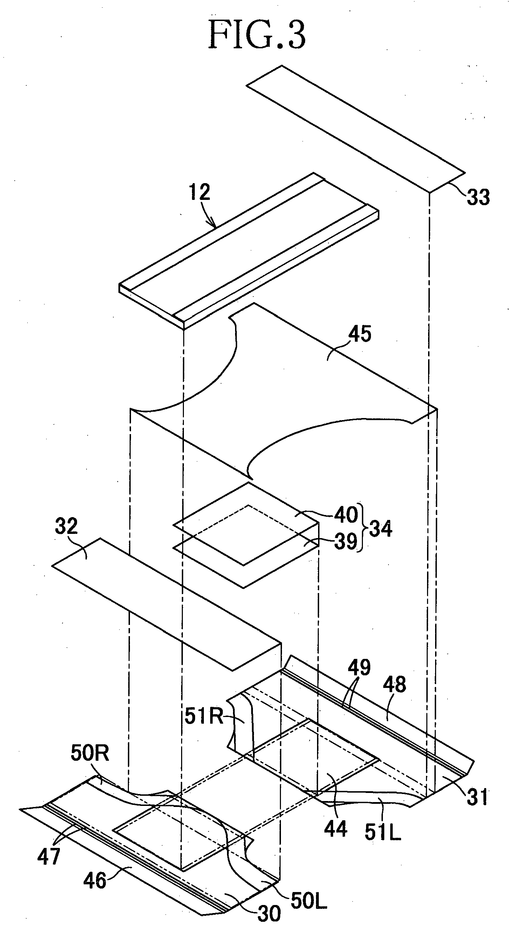

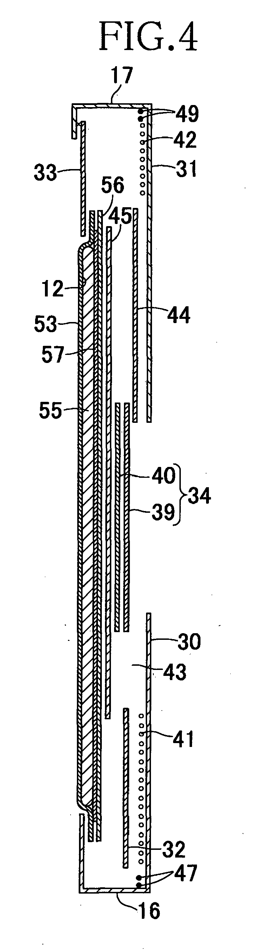

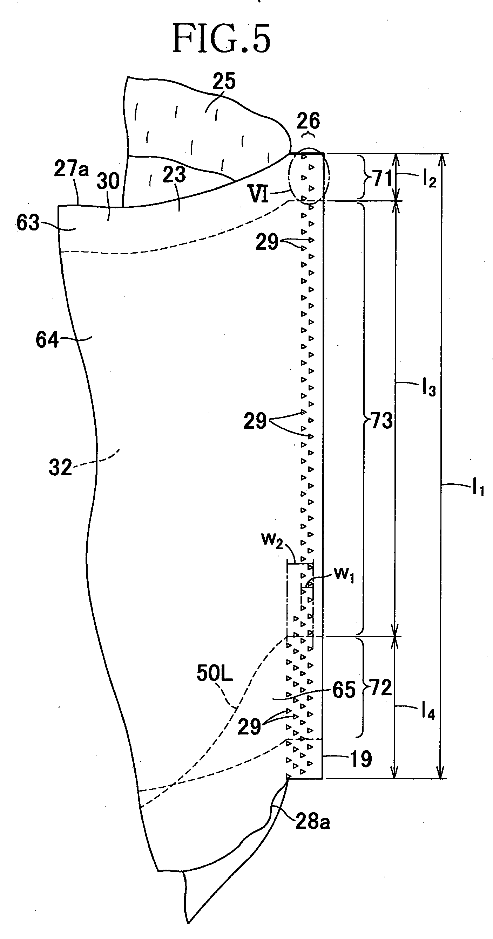

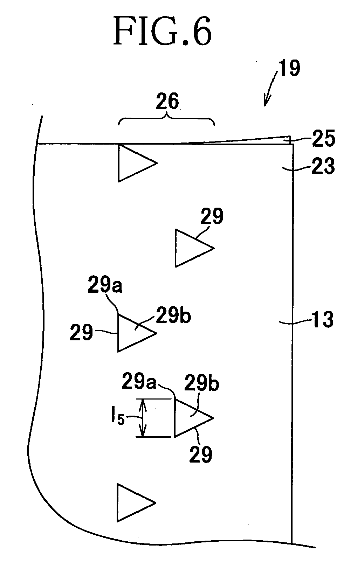

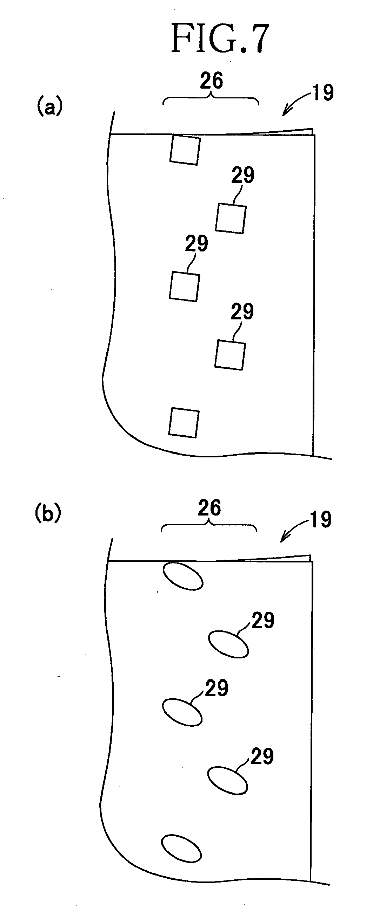

{Fig. 1} Fig. 1 is a perspective view of a disposable diaper as one example of pants-type disposable wearing article according to a first embodiment of the present invention.
{Fig. 2} Fig. 2 is a partially cutaway plan view showing the diaper as has been opened out and flattened.
{Fig. 3} Fig. 3 is an exploded perspective view of the diaper.
{Fig. 4} Fig. 4 is a schematic sectional view taken along line IV-IV in Fig. 2.
{Fig. 5} Fig. 5 is a partially enlarged diagram showing a region enclosed by a dashed-dotted line in Fig. 1.
{Fig. 6} Fig. 6 is a partially enlarged diagram showing a region enclosed by a dashed-dotted line in Fig. 5.
{Fig. 7} Figs. 7 (a)-(b) are diagrams similar to Fig. 6, showing second and third embodiments of the present invention.

### {Description of Embodiments}

### <First Embodiment>

Fig. 1 is a perspective view of a disposable diaper 10 as one example of the pants-type disposable wearing articles according to a first embodiment of the present invention, Fig. 2 is a partially cutaway plan view showing the diaper 10 as has been opened out and flattened, Fig. 3 is an exploded perspective view of the diaper 10 and Fig. 4 is a schematic sectional view taken along the line IV-IV in Fig. 2. In Fig. 2, a longitudinal axis of the diaper 10 is designated by P and a transverse axis thereof is designated by Q. The diaper 10 is shaped symmetrically about the longitudinal axis P.

The diaper 10 has a longitudinal direction Y, a transverse direction X being orthogonal thereto, a side facing a wearer's skin, a side facing away from the wearer's skin, a chassis 11 defining an outer shape of the diaper 10 and a liquid-absorbent structure 12 lying on the side of the chassis 11 facing the wearer's skin.

The diaper 10 has a front waist region 13, a rear waist region 14, a crotch region 15 extending between the front and rear waist regions 13, 14, front and rear ends 16, 17 opposed to each other in the longitudinal direction Y and extending in the transverse direction X and opposite side edges 18, 19 opposed to each other in the transverse direction X and extending in the longitudinal direction Y.

The opposite side edges 18, 19 are respectively concavely curved in the crotch region 15 so that these side edges 18, 19 may come in close contact with a wearer's thighs at a desired fit. Opposite side edges 22, 23 of the front waist region 13 which will be referred to hereinafter as front opposite side edges 22, 23 and opposite side edges 24, 25 of the rear waist region 14 which will be referred to hereinafter as rear opposite side edges 24, 25 partially constitute the opposite side edges 18, 19. The front side edges 22, 23 and the rear side edges 24, 25 respectively opposed in a front-back direction of the diaper 10 are joined together by the respective side seams 26 extending intermittently in the longitudinal direction Y to form a waist-opening 27 and a pair of leg-openings 28 (See Fig. 1). In each of the side seams 26, the sheets are put flat and fusion-bonded together by a plurality of concave sealing spots 29 formed by heat- or ultrasonic-embossing and intermittently arranged in the longitudinal direction Y.

The chassis 11 includes a substantially trapezoidal first outer sheet 30 lying on the non-skin-facing side and defining the front waist region 13, a substantially trapezoidal second outer sheet 31 defining the rear waist region 14, a first inner sheet 32 extending in the transverse direction X in the front waist region 13 and bonded to the side of the first outer sheet 30 facing the wearer's skin, a second inner sheet 33 extending in the transverse direction X in the rear waist region 14 and bonded to the side of the second outer sheet 31 facing the wearer's skin and a substantially rectangular intermediate sheet 34 extending between the first and second outer sheets 30, 31 so as to define a midportion of the crotch region 15.

Referring to Fig. 4, the first outer sheet 30 and the first inner sheet 32 are bonded to each other with hot melt adhesives 41 to form a first laminated sheet 37. The second inner sheet 33 has a width dimension smaller than that of the first inner sheet 32 and the second outer sheet 31 is bonded to the second inner sheet 33 with hot melt adhesives 42 to form a second laminated sheet 38. The intermediate sheet 34 includes a substantially rectangular fibrous nonwoven fabric sheet 39, lying on the non-skin-facing side, and a moisture-pervious but liquid-impervious plastic sheet 40, lying on the side facing the wearer's skin and being substantially the same as the fibrous nonwoven fabric sheet 39 in shape as well as in size, bonded to each other with hot melt adhesives (not shown).

The chassis 11 further includes a graphic film 44 made of a plastic material located in the central area of the rear waist region 14 as viewed in the transverse direction X and printed on its side facing away from the wearer's skin with graphics (not shown) or the like adapted to be visually recognized from the outside and a fixing sheet 45 formed of a fibrous nonwoven fabric extending on the side of the chassis 11 facing the wearer's skin across the crotch region 15 into the front and rear waist regions 13, 14. The fixing sheet 45 has a width dimension larger than that of the intermediate sheet 34 and covers, in the central area of the crotch region 15, the entire side of the intermediate sheet 34 facing the wearer's skin.

Along the front end 16 (i.e., a peripheral edge 27a of the waist-opening 27) of the front waist region 13, the first outer sheet 30 is folded inward to form a front turnback 46 within which two or more strand-like first waist elastic elements 47 made of elastomers are contractibly attached under tension. Along the rear end 17 (i.e., a peripheral edge 27a of the waist-opening 27) of the rear waist region 14, the second outer sheet 31 is folded inward to form a rear turnback 48 within which two or more strand-like second waist elastic elements 49 made of elastomers are contractibly attached under tension.

Along respective segments (i.e., peripheral edges 28a of the leg-openings 28) of the opposite side edges 18, 19 of the diaper 10 extending from the bottom of the front waist region 13 to the portions of the crotch region 15 lying adjacent to the front waist region 13, tape-like front leg elastic elements 50R, 50L made of elastomers are attached to the inner surface of the first outer sheet 30. In a similar fashion, along respective segments (i.e., peripheral edges 28a of the leg-openings 28) of the opposite side edges 18, 19 of the diaper 10 extending from the bottom of the rear waist region 14 to the portions of the crotch region 15 lying adjacent to the rear waist region 14, tape-like rear leg elastic elements 51R, 51L made of elastomers are attached to the inner surface of the second outer sheet 31. The rear leg elastic elements 51R, 51L and parts of the front leg elastic elements 50R, 50L are coated on respective outer surfaces thereof with hot melt adhesives (not shown) and attached to the fixing sheet 45 with the hot melt adhesives.

The first and second outer sheets 30, 31 are respectively formed of heat-sealable spun bonded nonwoven fabric having substantially no elastic stretch properties. This fibrous nonwoven fabric has an apparent thickness of about 0. 3 to 0.5mm, a mass per unit area of about 15 to about 40g/m², preferably about 25 to about 35g/m², a fiber density of about 0.06 to about 0.1g/cm³, preferably about 0.07 to about 0.09g/cm³. The first and second outer sheets 30, 31 may be formed of two or more layers and the respective outermost fiber layers may be formed of crimped spun bonded filament fibers. The first and second outer sheets 30, 31 may be formed of a fibrous nonwoven fabric having an elastic contraction percentage lower than that of the first and second outer sheets 32, 33.

The first and second inner sheets 32, 33 are respectively formed of an elastically stretchable air-through fiber (staple) nonwoven fabric or a spun bonded nonwoven fabric both made of heat-sealable elastomer fibers. Such nonwoven fabric has an apparent thickness of about 0.9 to about 1.0mm, a mass per unit area of about 20 to about 50g/m², preferably about 30 to about 40g/m² and a fiber density of about 0.01 to about 0.04g/cm³, preferably about 0.025 to about 0.035g/cm³.

The first and second outer sheets 30, 31 have a stretch ratio of about 100 to about 150% and the first and second inner sheets 32, 33 have a stretch ratio of about 150 to 300%. The first and second inner sheets 32, 33 are bonded under tension at a ratio of about 2.0 to about 3.0 to the first and second outer sheets 30, 31 with hot melt adhesives.

Hot melt adhesives 41, 42 used to bond the first and second outer sheets 30, 31 to the first and second inner sheets 32, 33 are not limited to any particular types and various types of adhesives widely used in the related technical field may be used without limitation so long as a desired elasticity for the first and second inner sheets 32, 33 is not affected. To this end, rubber-based adhesives such as SBS (styrene-butadiene-styrene)-adhesives or SIS (styrene-isoprene-styrene)-adhesives, each having a mass per unit area of about 1.0 to 5.0g/m² is preferably used for this purpose. Coating patterns for hot melt adhesives 41, 42 may be selected from various well known patterns such as a Q-pattern, a spiral pattern, a dotted pattern and a wavy pattern.

The intermediate sheet 34 and the fixing sheet 45 may be formed of a spun bonded fibrous nonwoven fabric and/or an inelastic air-through fibrous nonwoven fabric similarly to the first and second outer sheets 30, 31.

The liquid-absorbent structure 12 has a vertically long rectangular shape extending across the crotch region 15 into the front and rear waist regions 13, 14 contoured by front and rear ends and opposite side edges which are orthogonal to the front and rear ends. The liquid-absorbent structure 12 comprises a liquid-pervious inner sheet 53 lying on the side facing the wearer's skin, a liquid-absorbent core 55 formed of wrapping a liquid-absorbent material assembly including a mixture of fluff pulp fibers and superabsorbent polymer particles with a liquid-dispersant sheet (not shown), a cover sheet 56 lying on the non-skin-facing side so as to cover the entire liquid-absorbent core 55 from the non-skin-facing side and a leakage-barrier sheet 57 formed of a plastic material and sandwiched between the liquid-absorbent core 55 and the cover sheet 56.

The cover sheet 56 has opposite laterals extending outward in the transverse direction X beyond the opposite side edges of the liquid-absorbent core 55. These laterals are partially folded inward to form a pair of sleeve-like side flaps 58R, 58L extending in the direction of the longitudinal axis P. Within the respective side flaps 58R, 58L, four strand-like elastic elements 59, 60 made of elastomers and extending in the longitudinal direction Y are contractibly attached under tension with hot melt adhesives. Of these elastic elements 59, 60, the respective two elastic elements 59a, 60a, lying outwardly, are located in the middle of the crotch region 15 as viewed in the longitudinal direction Y and cooperate with the first and second leg elastic elements 50R, 50L, 51R, 51L to define elastic belt-like zones adapted to extend along the wearer's inguinal regions when the diaper 10 is put on the wearer's body. Of the elastic elements 59, 60, the respective two elastic elements 59b, 60b, lying inwardly, extend within the respective side flaps 58R, 58L across the crotch region 15 into the front and rear waist regions 13, 14. Under contraction of these elastic elements 59, 60, the opposite laterals of the cover sheet 56 are spaced upward from the inner sheet 53 to form barrier- or gasket-cuffs adapted to prevent sideways leakage of body waste.

The side of the liquid-absorbent structure 12 facing away from the wearer's skin is entirely or partially bonded to the side of the chassis 11 facing the wearer's skin with hot melt adhesives (not shown). A rear end of the liquid-absorbent structure 12 is sandwiched between the second inner sheet 33 and the second outer sheet 31 and thereby body waste should not leak out beyond the rear end of the liquid-absorbent structure 12.

Fig. 5 is a partially enlarged diagram showing a region enclosed by a dashed-dotted line in Fig. 1. In Fig. 5, the first inner sheet 32 and the first leg elastic element 50L are indicated by broken lines for convenience of explanation. The rear waist region 14 has a construction basically similar to that of the front waist region 13 and, in view of this, description will be made hereinafter with respect only to the front waist region 13.

For convenience of illustration, Fig. 5 illustrates a side seam 26 as if the side seam 26 is divided into a top segment 71 lying adjacent to the peripheral edges 27a of the waist-opening, a bottom segment 72 lying adjacent to the peripheral edge 28a of the leg-opening and an intermediate segment 73 extending between the top and bottom segments 71, 72.

The top segment 71 is formed in a non-elasticized region 63 defined by the front turnback 46 of the first outer sheet 30 along the side edge 19 of the diaper 10, the intermediate segment 73 is formed in a first elasticized region 64 in which the first outer sheet 30 and the elasticized first inner sheet 32 are present, and the bottom segment 72 is formed in a second elasticized region 65 in which the first inner and outer sheets 30, 32 overlap the first leg elastic element 50R. With regard to dimensions of the respective segments 71, 72, 73 as measured in the longitudinal direction Y, specifically on the assumption that a length l₁ of the side edge 19 of the diaper 10 as measured in the longitudinal direction Y is about 100mm (L-size of the standard infant diaper), a dimension l₂ of the top segment 71 ranges about 10 to 30mm, a dimension l₃ of the intermediate segment 73 ranges about 80 to 50mm, and a dimension l₄ of the bottom segment 72 ranges about 10 to 40mm.

As will be apparent from Fig. 5, the respective sealing spots 29 in the respective segments 71, 72, 73 are substantially the same in shape as well as in area and arranged intermittently in the longitudinal direction Y as well as in the transverse direction X. A distance between each pair of adjacent sealing spots in the longitudinal direction Y is larger in the top segment 71 than in the intermediate segment 73 but such a distance in the intermediate segment 73 is substantially the same as in the bottom segment 72. In this way, the bottom segment 72 and the intermediate segment 73 respectively have a given sealing area but a sealing area in the top segment 71 adjacent to the peripheral edge 27a of the waist-opening is smaller than those in the intermediate segment 73 and the bottom segment 72. In consequence, the side edge 19 can be easily torn from the top segment 71 when the used diaper 10 is removed from the wearer's body. The respective sealing spots 29 are arranged not contiguously one to another but intermittently in the longitudinal direction Y as well as in the transverse direction X, i.e., these sealing spots 29 are arranged in a zigzag pattern. With such an arrangement of the sealing spots 29, the side edge 19 of the diaper 10 is adapted to be torn zigzag in the longitudinal direction Y. Therefore, even if the top segment 71 or the bottom segment 72 is partially in an unsealed state, the side seam 26 should not be unintentionally unsealed over a wide range during use of the diaper 10.

Specifically, the distance between each pair of the adjacent sealing spots 29 in the top segment 71 as measured in the longitudinal direction Y is preferably about 2.0 to about 6.0mm and the distance between each pair of the adjacent sealing spots 29 in the intermediate segment 73 and the bottom segment 72 as measured in the longitudinal direction Y is preferably about 0.5 to about 4.0mm.

In the bottom segment 72, a dimension of the side seam 26 in the transverse direction X is larger than the top segment 71 and the intermediate segment 73 and, in this way, the sealing spots 29 are arranged to have a larger sealing area in comparison to the top segment 71 and the intermediate segment 73. More specifically, while the top segment 71 and the intermediate segment 73 respectively comprise two columns of the sealing spots 29 arranged in a zigzag, the bottom segment 72 comprises three or more columns of the sealing spots 29 inclusive of the above-mentioned two columns. By dimensioning the bottom segment 72 to be larger than the top segment 71, a substantial circumferential dimension of the diaper 10 adjacent to the peripheral edge 28a of the leg-opening can be made narrower than a substantial circumferential dimension of the diaper 10 adjacent to the peripheral edge 27a of the waist-opening. Consequentially, the diaper 10 comes in contact about the wearer's thighs with a fit sufficient to prevent body waste from leaking sideways and, at the same time, the waist-opening 27 ensures a sufficient dimension to facilitate the putting on of the diaper 10 on the wearer's body.

In the diaper 10 according to the present embodiment, a width dimension W₁ of the side seam in the top segment 71 and the intermediate segment 73 in the transverse direction X is about 0.5 to about 5.5mm and a width dimension W₂ of the side seam in the bottom segment 72 in the transverse direction X is about 3.0 to about 6.0mm. The sealing spots 29 in the segments 71, 72, 73 have area ratios preferably of about 3.4 to about 4.0%, about 7.5 to about 9.0% and 4.4 to about 5.0%, respectively, of a given width (6mm) of the non-elasticized region 63, the first elasticized region 64 and the second elasticized region 65.

The respective sealing spots 29 have a substantially uniform area and the bottom segment 72 has a width dimension larger than that of the top segment 71. Under such conditions, the bottom segment 72 will have a seam strength higher than that of the other segments 71, 73 so long as the top segment 71 and the bottom segment 72 comprise the sheets laminated under the same conditions. The bottom segment 72 extending adjacent to the peripheral edge 28a of the leg-opening would otherwise be apt to get wrinkled due to movements of the wearer's leg and to unseal the laminated sheets. However, the bottom segment 73 according to the present embodiment has a relatively high seam strength and the seam array should not be unintentionally unsealed. According to the present embodiment, the first leg elastic element 50L, made of elastic material such as urethane, is sandwiched between the first inner sheet 32 and the first outer sheet 30 and bonded thereto. Such arrangement would otherwise make it difficult to fusion bond the sheets under pressure and, in consequence, the seam strength would otherwise be lower in the bottom segment 72 than in the top segment 71. However, the width of the bottom segment 72 is dimensioned to be larger than that of the top segment 71 so that the sealing area may be larger in the bottom segment 72 than in the top segment 71 according to the present embodiment. In this way, the bottom segment 72 ensures sufficient seam strength to eliminate the possibility that this segment might be unsealed during use of the diaper 10.

Specifically, as a result of actual measurement using the tensile tester of known art, seam strength per unit area (1 inch) was 10 to 25N/inch in the top segment 71, 8 to 18N/inch in the intermediate segment 73 and 10 to 35N/inch in the bottom segment 72.

The bottom segment 72 is shaped so as to extend obliquely inward from the intermediate segment 73 so that the width of the segment 73 may be gradually enlarged as the segment 72 slopes down inwardly. More specifically, a dimension of the innermost column in the bottom segment 72 as viewed in the longitudinal direction Y is smaller than that of the column immediately adjacent thereto and the dimension of this adjacent column in the longitudinal direction Y is smaller than that of further outer two columns (defining the top segment 71 and the intermediate segment 73). In this manner, the bottom segment 72 is stepwisely configured as a whole.

The bottom segment 72 obliquely extends inward from the intermediate segment 73, facilitating the bottom segment 72 to be torn. In addition, should any amount of body waste flow into the front waist region 13, such amount of body waste can be quickly moved onto the liquid-absorbent structure 12 without the possibility that the amount of body waste might be accumulated on the step defined between the intermediate segment 73 and the bottom segment 72.

Fig. 6 is a partially enlarged diagram showing a region enclosed by a dashed-dotted line in Fig. 5, and Figs. 7(a)-(b) are diagrams similar to Fig. 6, showing second and third embodiments of the present invention. Both the second embodiment and the third embodiment are similar to the first embodiment so long as the basic construction is concerned and therefore the description given hereinafter will be limited to features of these second and third embodiments differing from those of the first embodiment.

Referring to Fig. 6, in the lateral 19 of the diaper 10, each of the sealing spots 29 has a substantially triangular shape arranged obliquely to the longitudinal axis P of the diaper 10. The sealing spots 29 are arranged intermittently in the direction of the longitudinal axis P as well as in the direction of the transverse axis Q and obliquely to the longitudinal axis P of the diaper 10. Each of the sealing spots 29 is arranged so that its area may be gradually enlarged downwardly (i.e., toward the base) from the upper corner 29a (i.e., apex angle). With such a posture of the individual sealing spot 29, the force acting to tear the respective sealing spots 29 which is generated when the front and rear waist regions 13, 14 are pulled apart in the front-back direction of the diaper 10 by gripping the side edges 23, 25 of the front and rear waist regions 13, 14 is first locally exerted on the upper corner 29a and this force can be smoothly transmitted to a region 29b having an area gradually enlarged downwardly. Therefore, even when the first and second outer sheets 30, 31 defining the top segment 71 are the sheets formed of long fibers and more difficult to be torn in the direction of the longitudinal axis P than the sheets formed of short fibers, the respective sealing spots 29 can be torn by relatively moderate force. The shape of the sealing spot 29 is not limited to the shape as illustrated in Fig. 6 so long as the above-mentioned feature is ensured. For example, the sealing spot 29 may be substantially square as illustrated in Fig. 7(a) or substantially circular as illustrated in Fig. 7(b).

Each of the sealing spots 29 is relatively small and specifically has an area of about 0.25 to about 2.0mm. The smaller the size of the sealing spot 29 is, the smaller force there is needed to tear the sealing spot 29. The above-mentioned area of the individual sealing spot 29 is preferable for this reason.

While a dimension l₅ of the individual sealing spot 29 in the longitudinal direction Y (i.e., seal dimension) may be appropriately set depending on the seam strength required for each of the segments 71, 72, 73, it is also possible to set the seal dimension l₅ to become larger from the top segment 71 toward the bottom segment 72 so that the seam strength may gradually increase from the top segment 71 toward the bottom segment 72. In this case, the seal dimension l₅ of the individual sealing spot 29 in the top segment 71 preferably is about 0.5 to about 5.5mm and the seal dimension l₅ of the individual sealing spot 29 in the intermediate segment 73 and the bottom segment 72 is preferably about 3.0 to 6.0mm.

### {Reference Signs List}

- 10: pants-type disposable wearing article
- 13: front waist region
- 14: rear waist region
- 15: crotch region
- 18, 19: opposite side edges of diaper
- 26: side seam
- 27: waist-opening
- 29: sealing regions
- 30, 31: outer sheets
- 32, 33: inner sheets
- 59R, 50L: front leg elastic elements
- 51R, 51L: rear leg elastic elements
- 63: non-elasticized region
- 64: first elasticized region
- 65: second elasticized region
- 71: top segment
- 72: bottom segment
- 73: intermediate segment

## Claims

1. A pants-type disposable wearing article (10) having a longitudinal axis and a transverse axis being orthogonal thereto, comprising:
a chassis (11) defining an outer shape of the disposable wearing article;
a liquid-absorbent structure (12) lying on the side of the chassis facing the wearer's skin
a front waist region (13);
a rear waist region (14);
a crotch region (15) extending between the front and rear waist regions;
front and rear ends (16, 17) opposed to each other in the longitudinal direction and extending in the transverse direction;
opposite side edges (18, 19) opposed to each other in the transverse direction and extending in the longitudinal direction, wherein the opposite side edges are respectively concavely curved in the crotch region;
a substantially trapezoidal non-elasticized first outer sheet (30) lying on the non-skin-facing side and defining the front waist region;
a substantially trapezoidal non-elasticized second outer sheet (31) defining the rear waist region;
a first elasticized inner sheet (32) extending in the transverse direction in the front waist region and bonded to the side of the first outer sheet facing the wearer's skin;
a second elasticized inner sheet (33) extending in the transverse direction in the rear waist region and bonded to the side of the second outer sheet facing the wearer's skin;
a substantially rectangular intermediate sheet (34) extending between the first and second outer sheets so as to define a midportion of the crotch region;
a graphic film (44) made of a plastic material located in the central area of the rear waist region as viewed in the transverse direction and printed on its side facing away from the wearer's skin with graphics or the like adapted to be visually recognized from the outside;
a fixing sheet (45) formed of a fibrous nonwoven fabric extending on the side of the chassis facing the wearer's skin across the crotch region into the front and rear waist regions; wherein the fixing sheet has a width dimension larger than that of the intermediate sheet and covers, in the central area of the crotch region, the entire side of the intermediate sheet facing the wearer's skin;
tape-like front leg elastic elements (50R, 50L) along respective segments of the opposite side edges of the diaper extending from the bottom of the front waist region to the portions of the crotch region lying adjacent to the front waist region; the tape-like front leg elastic elements being made of elastomers and being attached to the inner surface of the first outer sheet by being sandwiched between the first inner sheet (32) and the first outer sheet (30) and bonded thereto;
tape-like rear leg elastic elements (51R, 51L) along respective segments of the opposite side edges of the diaper extending from the bottom of the rear waist region to the portions of the crotch region lying adjacent to the rear waist region, the tape-like rear leg elastic elements being made of elastomers and being attached to the inner surface of the second outer sheet;
the rear leg elastic elements and parts of the front leg elastic elements being coated on respective outer surfaces thereof with hot melt adhesives and attached to the fixing sheet with the hot melt adhesives;
a waist-opening (27);
a pair of leg-openings (28); and
side seams (26) by which the front and rear waist regions are joined together along opposite side edges thereof, wherein:
the side seams are respectively formed of a plurality of columns of sealing spots (29), the columns being spaced apart from one another in a direction along the transverse axis and the sealing spots (29) being arranged intermittently in a direction along the longitudinal axis and in the transverse direction such that the sealing spots are arranged in a zigzag pattern;
along the front end (16) of the front waist region (13), the first outer sheet (30) is folded inward to form a front turnback (46) within which two or more strand-like first waist elastic elements (47) made of elastomers are contractibly attached under tension;
along the rear end (17) of the rear waist region (14), the second outer sheet (31) is folded inward to form a rear turnback (48) within which two or more strand-like second waist elastic elements (49) made of elastomers are contractibly attached under tension;
the opposite side edges of the front and rear waist regions (13,14) respectively include non-elasticized regions (63) formed of the first and second outer sheets respectively; first elasticized regions (64) lying below the non-elasticized regions (63) and formed of the first and second non-elasticized outer sheets respectively and the first and second elasticized inner sheets respectively; and second elasticized regions (65) lying below the first elasticized regions (64) and formed of the first and second outer sheets respectively, the first and second inner sheets respectively and the tape-like front and rear leg elastic elements respectively;
each of the side seams (26) comprises a top segment (71) located adjacent to the waist-opening, a bottom segment (72) located adjacent to the crotch region and an intermediate segment (73) extending between the top segment and the bottom segment;
the top segments are formed in the non-elasticized regions (63), the intermediate segments are formed in the first elasticized regions (64) and the bottom segments are formed in the second elasticized regions (65);
a distance between each pair of the adjacent sealing spots in the longitudinal direction is larger in the top segment than in the intermediate segment but such a distance in the intermediate segment is substantially the same as in the bottom segment;
the top segment and the intermediate segment respectively comprise two columns of the sealing spots and the bottom segment comprises three or more columns of the sealing spots inclusive of the above-mentioned two columns;
each of the sealing spots has a substantially uniform area; and
a dimension (w2) of the bottom segment in a direction along the transverse axis is larger than a dimension (w1) of the top segment and the intermediate segment in the direction along the transverse axis.

2. The wearing article according to claim 1, wherein a dimension of a side seam array in the direction along the longitudinal axis is gradually reduced in the bottom segment of the side seams from the outside toward the inside as viewed in the direction along the transverse axis.

3. The wearing article according to claim 1or 2, wherein the sealing spots are arranged obliquely to the longitudinal axis and shaped so that a width dimension of the sealing spots is gradually enlarged in a direction of top-to-bottom.

## Patentansprüche

1. Höschenähnlicher Einwegbekleidungsartikel (10), der eine Längsachse und eine Querachse, die dazu senkrecht vorliegt, aufweist, der Folgendes umfasst:
einen Grundkörper (11), der eine äußere Form des Einwegbekleidungsartikels definiert;
eine flüssigkeitsabsorbierende Struktur (12), die auf der Seite des Grundkörpers aufliegt, die der Haut des Trägers zugewandt ist;
einen vorderen Taillenbereich (13);
einen hinteren Taillenbereich (14);
einen Schrittbereich (15), der sich zwischen dem vorderen und hinteren Taillenbereich erstreckt;
vorderes und hinteres Ende (16, 17), die in der Längsrichtung einander gegenüberliegen und sich in der Querrichtung erstrecken;
gegenüberliegende Seitenränder (18, 19), die in der Querrichtung einander gegenüberliegen und sich in der Längsrichtung erstrecken, wobei die gegenüberliegenden Seitenränder in dem Schrittbereich jeweils konkav gewölbt sind;
eine im Wesentlichen trapezförmige nichtelastische erste äußere Lage (30), die auf der Seite, die nicht der Haut zugewandt ist, aufliegt und den vorderen Taillenbereich definiert;
eine im Wesentlichen trapezförmige nichtelastische zweite äußere Lage (31), die den hinteren Taillenbereich definiert;
eine erste elastische innere Lage (32), die sich in der Querrichtung in dem vorderen Taillenbereich erstreckt und zu der Seite der ersten äußeren Lage, die der Haut des Trägers zugewandt ist, gebunden ist;
eine zweite elastische innere Lage (33), die sich in der Querrichtung in dem hinteren Taillenbereich erstreckt und zu der Seite der zweiten äußeren Lage, die der Haut des Trägers zugewandt ist, gebunden ist;
eine im Wesentlichen rechteckige Zwischenlage (34), die sich zwischen der ersten und zweiten äußeren Lage erstreckt, sodass ein Mittelabschnitt des Schrittbereichs definiert wird;
eine grafische Folie (44), die aus einem Kunststoffmaterial hergestellt ist, das sich in dem mittleren Bereich des hinteren Taillenbereichs, in Querrichtung gesehen, befindet und auf dessen Seite, die von der Haut des Trägers abgewandt ist, mit Grafiken oder dergleichen bedruckt ist, die geeignet sind, von außen visuell erkannt zu werden;
eine befestigende Lage (45), die aus einem Faservlies gebildet ist, das sich an der Seite des Grundkörpers, die der Haut des Trägers zugewandt ist, über den Schrittbereich in den vorderen und hinteren Taillenbereich erstreckt;
wobei die befestigende Lage eine Breitenabmessung aufweist, die größer ist als die der Zwischenlage, und in dem mittleren Bereich des Schrittbereichs die gesamte Seite der Zwischenlage, die der Haut des Trägers zugewandt ist, bedeckt;
bandartige vordere elastische Elemente für das Bein (50R, 50L) entlang den entsprechenden Segmenten der gegenüberliegenden Seitenränder der Windel, die sich von der Unterseite des vorderen Taillenbereichs zu den Abschnitten des Schrittbereichs, die benachbart zum vorderen Taillenbereich liegen, erstrecken; wobei die bandartigen vorderen elastischen Elemente für das Bein aus Elastomeren hergestellt sind und an der inneren Oberfläche der ersten äußeren Lage durch Einfügung zwischen die erste innere Lage (32) und die erste äußere Lage (30) und Bindung daran angebracht sind;
bandartige hintere elastische Elemente für das Bein (51R, 51L) entlang den entsprechenden Segmenten der gegenüberliegenden Seitenränder der Windel, die sich von der Unterseite des hinteren Taillenbereichs zu den Abschnitten des Schrittbereichs, die benachbart zum hinteren Taillenbereich liegen, erstrecken, wobei die bandartigen hinteren elastischen Elemente für das Bein aus Elastomeren hergestellt sind und an der inneren Oberfläche der zweiten äußeren Lage angebracht sind;
wobei die hinteren elastischen Elemente für das Bein und Teile der vorderen elastischen Elemente für das Bein an den entsprechenden äußeren Oberflächen davon mit Schmelzklebstoffen beschichtet und an die befestigende Lage mit den Schmelzklebstoffen angebracht sind;
eine Taillenöffnung (27);
ein Paar an Beinöffnungen (28); und
Seitennähte (26), durch die der vordere und hintere Taillenbereich entlang gegenüberliegenden Seitenränder davon miteinander verbunden sind, wobei:
die Seitennähte jeweils aus einer Vielzahl von Säulen von Abdichtungspunkten (29) gebildet sind, wobei die Säulen in einer Richtung entlang der Querachse voneinander beanstandet sind und die Abdichtungspunkte (29) in einer Richtung entlang der Längsachse und in der Querrichtung intermittierend angeordnet sind, sodass die Abdichtungspunkte in einem Zickzackmuster angeordnet sind;
entlang des vorderen Endes (16) des vorderen Taillenbereichs (13) die erste äußere Lage (30) nach innen gefaltet ist, um einen vorderen Umschlag (46) zu bilden, in dem zwei oder mehr strangartige erste elastische Elemente der Taille (47), die aus Elastomeren hergestellt sind, unter Spannung zusammenziehbar angebracht sind;
entlang des hinteren Endes (17) des hinteren Taillenbereichs (14) die zweite äußere Lage (31) nach innen gefaltet ist, um einen hinteren Umschlag (48) zu bilden, in dem zwei oder mehr strangartige zweite elastische Elemente der Taille (49), die aus Elastomeren hergestellt sind, unter Spannung zusammenziehbar angebracht sind;
die gegenüberliegenden Seitenränder des vorderen und hinteren Taillenbereichs (13, 14) jeweils nichtelastische Bereiche (63) einschließen, die aus der ersten bzw. zweiten äußeren Lage gebildet sind; wobei erste elastische Bereiche (64) unter den nichtelastischen Bereichen (63) liegen und aus der ersten bzw. zweiten nichtelastischen äußeren Lage und der ersten bzw. zweiten elastischen inneren Lage gebildet sind; und zweite elastische Bereiche (65) unter den ersten elastischen Bereichen (64) liegen und aus der ersten bzw. zweiten äußeren Lage, der ersten bzw. zweiten inneren Lage und den bandartigen vorderen bzw. hinteren elastischen Elementen für das Bein gebildet sind;
jede der Seitennähte (26) ein oberes Segment (71), das sich benachbart zur Taillenöffnung befindet, ein unteres Segment (72), das sich benachbart zum Schrittbereich befindet, und ein Zwischensegment (73), das sich zwischen dem oberen Segment und dem unteren Segment erstreckt, umfasst;
die oberen Segmente in den nichtelastischen Bereichen (63) ausgebildet sind, die Zwischensegmente in den ersten elastischen Bereichen (64) ausgebildet sind und die unteren Segmente in den zweiten elastischen Bereichen (65) ausgebildet sind;
ein Abstand zwischen jedem Paar der benachbarten Abdichtungspunkte in der Längsrichtung in dem oberen Segment größer ist als in dem Zwischensegment, doch ein derartiger Abstand in dem Zwischensegment im Wesentlichen der gleiche ist wie in dem unteren Segment;
das obere Segment und das Zwischensegment jeweils zwei Säulen der Abdichtungspunkte umfassen und das untere Segment drei oder mehr Säulen der Abdichtungspunkte, einschließlich der vorstehend erwähnten zwei Säulen, umfasst;
jeder der Abdichtungspunkte eine im Wesentlichen gleichmäßige Fläche aufweist; und
eine Abmessung (w2) des unteren Segments in einer Richtung entlang der Querachse größer ist als eine Abmessung (w1) des oberen Segments und des Zwischensegments in der Richtung entlang der Querachse.

2. Bekleidungsartikel nach Anspruch 1, wobei sich eine Abmessung einer Seitennahtanordnung in der Richtung entlang der Längsachse in dem unteren Segment der Seitennähte von außen nach innen, in der Richtung entlang der Querachse gesehen, allmählich verringert.

3. Bekleidungsartikel nach Anspruch 1 oder 2, wobei die Abdichtungspunkte schräg zur Längsachse angeordnet und geformt sind, sodass sich eine Breitenabmessung der Abdichtungspunkte in einer Richtung von oben nach unten allmählich vergrößert.

## Revendications

1. Article portable jetable de type culotte (10) ayant un axe longitudinal et un axe transversal qui est perpendiculaire par rapport à celui-ci, comportant :
une armature (11) définissant une forme extérieure de l'article portable jetable ;
une structure absorbant les liquides (12) reposant sur le côté de l'armature orienté vers la peau de l'utilisateur,
une région avant au niveau de la taille (13) ;
une région arrière au niveau de la taille (14) ;
une région au niveau de l'entrejambe (15) s'étendant entre les régions avant et arrière au niveau de la taille ;
des extrémités avant et arrière (16, 17) opposées l'une par rapport à l'autre dans la direction allant dans le sens longitudinal et s'étendant dans la direction allant dans le sens transversal ;
des bords latéraux opposés (18, 19) opposés l'un par rapport à l'autre dans la direction allant dans le sens transversal et s'étendant dans la direction allant dans le sens longitudinal, dans lequel les bords latéraux opposés sont respectivement courbes de manière concave dans la région au niveau de l'entrejambe ;
une première feuille extérieure non élasticisée sensiblement trapézoïdale (30) reposant sur le côté non orienté vers la peau et définissant la région avant au niveau de la taille ;
une deuxième feuille extérieure non élasticisée sensiblement trapézoïdale (31) définissant la région arrière au niveau de la taille ;
une première feuille intérieure élasticisée (32) s'étendant dans la direction allant dans le sens transversal dans la région avant au niveau de la taille et liée au côté de la première feuille extérieure orienté vers la peau de l'utilisateur ;
une deuxième feuille intérieure élasticisée (33) s'étendant dans la direction allant dans le sens transversal dans la région arrière au niveau de la taille et liée au côté de la deuxième feuille extérieure orienté vers la peau de l'utilisateur ;
une feuille intermédiaire sensiblement rectangulaire (34) s'étendant entre les première et deuxième feuilles extérieures de manière à définir une partie médiane de la région au niveau de l'entrejambe ;
un film graphique (44) réalisé en une matière plastique se trouvant dans la zone centrale de la région arrière au niveau de la taille quand on observe dans la direction allant dans le sens transversal et imprimé sur son côté orienté à l'opposé de la peau de l'utilisateur avec des graphiques ou similaires adaptés pour être reconnus visuellement depuis l'extérieur ;
une feuille de fixation (45) formée sur un tissu non tissé fibreux s'étendant sur le côté de l'armature orienté vers la peau de l'utilisateur en travers de la région au niveau de l'entrejambe jusque dans les régions avant et arrière au niveau de la taille ; dans lequel la feuille de fixation a une dimension de largeur supérieure à celle de la feuille intermédiaire et recouvre, dans la zone centrale de la région au niveau de l'entrejambe, la totalité du côté de la feuille intermédiaire orienté vers la peau de l'utilisateur ;
des éléments élastiques pour jambe avant similaires à du ruban (50R, 50L) le long de segments respectifs des bords latéraux opposés de la couche s'étendant depuis le bas de la région avant au niveau de la taille jusqu'aux parties de la région au niveau de l'entrejambe reposant de manière adjacente par rapport à la région avant au niveau de la taille ; les éléments élastiques pour jambe avant similaires à du ruban étant réalisés à partir d'élastomères et étant attachés à la surface intérieure de la première feuille extérieure en étant pris en sandwich entre la première feuille intérieure (32) et la première feuille extérieure (30) et liés à celles-ci ;
des éléments élastiques pour jambe arrière similaires à du ruban (51R, 51L) le long de segments respectifs des bords latéraux opposés de la couche s'étendant depuis le bas de la région arrière au niveau de la taille jusqu'aux parties de la région au niveau de l'entrejambe reposant de manière adjacente par rapport à la région arrière au niveau de la taille, les éléments élastiques pour jambe arrière similaires à du ruban étant réalisés à partir d'élastomères et étant attachés à la surface intérieure de la deuxième feuille extérieure ;
les éléments élastiques pour jambe arrière et des parties des éléments élastiques pour jambe avant étant revêtus sur des surfaces extérieures respectives de ceux-ci d'adhésifs thermofusibles et attachés à la feuille de fixation au moyen des adhésifs thermofusibles ;
une ouverture pour la taille (27) ;
une paire d'ouvertures pour les jambes (28) ; et
des coutures latérales (26) par lesquelles les régions avant et arrière au niveau de la taille sont jointes ensemble le long de bords latéraux opposés de celles-ci, dans lequel :
les coutures latérales sont respectivement formées à partir d'une pluralité de colonnes de points de soudage (29), les colonnes étant espacées les unes des autres dans une direction allant le long de l'axe transversal et les points de soudage (29) étant agencés de manière intermittente dans une direction allant le long de l'axe longitudinal et dans la direction allant dans le sens transversal de telle sorte que les points de soudage sont agencés selon une configuration en zigzag ;
le long de l'extrémité avant (16) de la région avant au niveau de la taille (13), la première feuille extérieure (30) est pliée vers l'intérieur pour former un repli avant (46) dans lequel deux ou plusieurs premiers éléments élastiques au niveau de la taille similaires à des mèches (47) réalisés à partir d'élastomères sont attachés par contraction sous tension ;
le long de l'extrémité arrière (17) de la région arrière au niveau de la taille (14), la deuxième feuille extérieure (31) est pliée vers l'intérieur pour former un repli arrière (48) dans lequel deux ou plusieurs deuxièmes éléments élastiques au niveau de la taille similaires à des mèches (49) réalisés à partir d'élastomères sont attachés par contraction sous tension ;
les bords latéraux opposés des régions avant et arrière au niveau de la taille (13, 14) comprennent respectivement des régions non élasticisées (63) formées à partir des première et deuxième feuilles extérieures respectivement ; des premières régions élasticisées (64) reposant sous les régions non élasticisées (63) et formées à partir des première et deuxième feuilles extérieures non élasticisées respectivement et des première et deuxième feuilles intérieures élasticisées respectivement ; et des deuxièmes régions élasticisées (65) reposant sous les premières régions élasticisées (64) et formées à partir des première et deuxième feuilles extérieures respectivement, des première et deuxième feuilles intérieures respectivement et des éléments élastiques pour jambe avant et arrière similaires à du ruban respectivement ;
chacune des coutures latérales (26) comporte un segment supérieur (71) situé de manière adjacente par rapport à l'ouverture pour la taille, un segment inférieur (72) situé de manière adjacente par rapport à la région au niveau de l'entrejambe et un segment intermédiaire (73) s'étendant entre le segment supérieur et le segment inférieur ;
les segments supérieurs sont formés dans les régions non élasticisées (63), les segments intermédiaires sont formés dans les premières régions élasticisées (64) et les segments inférieurs sont formés dans les deuxièmes régions élasticisées (65) ;
une distance entre chaque paire de points de soudage adjacents dans la direction allant dans le sens longitudinal est supérieure dans le segment supérieur que dans le segment intermédiaire mais une telle distance dans le segment intermédiaire est sensiblement la même que dans le segment inférieur ;
le segment supérieur et le segment intermédiaire comportent respectivement deux colonnes des points de soudage et le segment inférieur comporte trois colonnes ou plus des points de soudage y compris les deux colonnes mentionnées ci-dessus ;
chacun des points de soudage a une superficie sensiblement uniforme ; et
une dimension (w2) du segment inférieur dans une direction allant le long de l'axe transversal est supérieure à une dimension (w1) du segment supérieur et du segment intermédiaire dans la direction allant le long de l'axe transversal.

2. Article portable selon la revendication 1, dans lequel une dimension d'une série de coutures latérales dans la direction allant le long de l'axe longitudinal est progressivement réduite dans le segment inférieur des coutures latérales depuis l'extérieur vers l'intérieur quand on observe dans la direction allant le long de l'axe transversal.

3. Article portable selon la revendication 1 ou la revendication 2, dans lequel les points de soudage sont agencés de manière oblique par rapport à l'axe longitudinal et façonnés de telle sorte qu'une dimension de largeur des points de soudage est progressivement agrandie dans une direction allant de haut en bas.
